(19) Europäisches Patentamt

European Patent Office

Office européen des brevets

(11) **EP 0 835 124 B1**

(12) **EUROPEAN PATENT SPECIFICATION**

(45) Date of publication and mention
of the grant of the patent:
**11.02.2004 Bulletin 2004/07**

(51) Int Cl.[7]: **A61K 38/00**, A61K 38/16,
A61K 39/395, A61P 35/00,
A61P 31/14, C07K 14/435,
C07K 16/06, C07K 16/10,
C07K 16/30

(21) Application number: **96920129.2**

(22) Date of filing: **03.05.1996**

(86) International application number:
**PCT/US1996/006245**

(87) International publication number:
**WO 1996/034616 (07.11.1996 Gazette 1996/49)**

(54) **SERUM PREPARATIONS FOR INHIBITING RETROVIRUSES AND TUMOR GROWTH**

SERUMZUSAMMENSETZUNGEN ZUR HEMMUNG VON RETROVIREN UND
TUMORWACHSTUM

PREPARATIONS DE SERUM VISANT A INHIBER LES RETROVIRUS ET LA CROISSANCE DE
TUMEURS

(84) Designated Contracting States:
**AT BE CH DE DK ES FI FR GB GR IE IT LI LU MC
NL PT SE**
Designated Extension States:
**AL LT LV SI**

(30) Priority: **03.05.1995 US 434438
10.10.1995 US 5133**

(43) Date of publication of application:
**15.04.1998 Bulletin 1998/16**

(73) Proprietor: **Viro Tech Limited Partnership
Sedro Woolley, WA 98284 (US)**

(72) Inventor: **HOWARD, Mark, E.
Everett, WA 98205 (US)**

(74) Representative:
**Gowshall, Jonathan Vallance et al
FORRESTER & BOEHMERT
Pettenkoferstrasse 20-22
80336 München (DE)**

(56) References cited:
- **M.M. SIGEL ET AL.: "STUDIES ON
IMMUNOGLOBULINS REACTIVE WITH TUMOR
CELLS AND ANTIGENS." CANCER RESEARCH,
vol. 28, no. 8, July 1968 (1968-07), pages
1457-1459, XP000938903 BALTIMORE, MD, US**
- **M.J.SNODGRASS ET AL.: "INHIBITORY EFFECT
OF SHARK SERUM ON THE LEWIS LUNG
CARCINOMA" JOURNAL OF THE NATIONAL
CANCER INSTITUTE, vol. 56, no. 5, May 1976
(1976-05), pages 981-984, XP000938862
WASHINGTON, DC, US**
- **DATABASE WPI Section Ch, Week 199521
Derwent Publications Ltd., London, GB; Class
B04, AN 1995-159703 XP002146109 & RU 2 019
180 C (BEZMAN YA D), 15 September 1994
(1994-09-15)**
- **CLINICAL AND EXPERIMENTAL IMMUNOLOGY,
1992, Vol. 88, FAHEY et al., "Status of
Immune-Based Therapies in HIV Infection and
AIDS", pages 1-5.**
- **BIO/TECHNOLOGY, February 1994, Vol. 12, FOX
J.L., "No Winners Against AIDS", page 128.**
- **GENES AND CANCER, CARNEY et al.,
CHICHESTER: JOHN WILEY & SONS LTD, 1990.**

**Description**

Background of the Invention

The disease acquired immunodeficiency syndrome, or AIDS, remains substantially refractory to therapy. Despite intensive efforts to develop compounds that inhibit the virus that causes the disease, HIV, the infection almost uniformly progresses and the individual's immune system is rendered dysfunctional. Infected patients become extremely susceptible to secondary diseases, such as pneumonia and Kaposi's sarcoma, which are often life-threatening. While drugs such as 3'-azido-3'-deoxythymidine (AZT), 2',3'-dideoxyinosine (ddI) and 2',3'-dideoxycytosine (ddC) have been approved for use in infected individuals, profound toxicities and the emergence of drug-resistant viral strains are associated with their use. There remains a critical need to identify new anti-retroviral agents for use alone or in combination with other antiviral agents.

Effective anti-retroviral therapy depends on identifying antiviral agents devoid of significant toxicity and which, when employed in therapy, do not readily result in the emergence of drug-resistant viral isolates. Compounds potentially useful in inhibiting HIV and other retroviral infections are screened in a number of systems. Initially, screening is conducted in in vitro models of susceptible cell lines. Animal models are then used to identify those compounds with anti-retroviral activity in vivo as well as possessing acceptable levels of host toxicity. The models preferably assess activity against retroviral viremia as well as ability to suppress retroviral-induced disease, such as the destruction of the immune system and central nervous system disease in the case of HIV.

One model which has been found to be particularly useful as a model of retroviral infection, including HIV infection, is the Rauscher leukemia virus (RLV) infection of mice. RLV is infectious and pathogenic in adult mice, and it is erythrotropic, causing a splenomegaly that is proportional to viral titer. Chirigos, Cancer Res. 24: 1035-1041 (1964). Shortly after inoculation abnormal spleen colonies are formed whose numbers reflect viral titers. Each colony is the result of a successful viral "hit," and the colony continues to enlarge while new target cells are being transformed continuously during the viremia. RLV also induces B-cell neoplasms. Thus, RLV infection results in a massive splenomegaly and erythroleukemia which kills infected animals within 4-5 weeks after inoculation. Weiss, Teich, Varmus, et al., RNA Tumor Viruses, 2d ed., Cold Spring Harbor Laboratory Press, pp 78-79, Cold Spring Harbor, NY (1984). RLV infection in mice has also been shown to reproduce certain immunological aspects of HIV infection in humans. See, e.g., Gabrilovich et al., Immunology 82: 82-87 (1994) and Gabrilovich et al., Eur. J. Immunol. 23:2932-2938 (1993).

The use of the RLV model to identify and evaluate anti-HIV agents has become widespread. For example, RLV as a model of HIV infection has been reported for evaluating AZT (Ruprecht et al., Nature 323: 467-469 (1986); Ruprecht, Intervirol. 30(S1): 2-11 (1989)), new lipophilic derivatives of AZT (Schwendener et al., Antiviral Res. 24: 79-93 (1994)), derivatives of tetrahydroimidazole[4,5,1-jk][1,4]-benzodiazepin-2(1H)-thione (Buckheit et al., AIDS Res. Human Retrovir. 9: 1097-1106 (1993), biological response modifiers, including tests conducted by the U.S. Food and Drug Administration, as reported for, e.g., poly [I,C]-LC, MVE-2 and CL 246,738 (Black et al., Annl. N.Y. Acad. Sci. 685: 467-470 (1993)), and combination anti-HIV therapies (e.g., Ruprecht, supra, Buckheit et al., supra, and Black et al., supra). And, because RLV induces leukemia in infected animals, the RLV model is also used extensively as a model for treating various types of cancers, particularly leukemias. Sydow and Wunderlich, Cancer Lett. 82: 89-94 (1994).

New therapeutic modalities are urgently needed to provide more effective treatments for inhibiting retroviral infection. Also needed are effective means to inhibit development of cancers, such as leukemias or other neoplasms. Compositions useful for these purposes should be relatively easy to prepare and administer, relatively non-toxic, and effective inhibitors of retroviral infection or particular neoplasms. Quite surprisingly, the present invention addresses these and other related needs.

Summary of the Invention

The present invention provides, in one embodiment, methods for preparing shark serum concentrate that are useful in inhibiting retroviral and neoplastic disease in a susceptible mammalian cell. Generally, the shark serum is produced by an admixture of ethanol and shark blood, followed by removal of the ethanol, typically by heating to a temperature and for a time sufficient to evaporate the ethanol but not sufficient to inactivate serum proteins such as immunoglobulin and complement. The serum is then separated from any precipitate and is used directly or to form compositions, including pharmaceutical compositions, useful for inhibiting retroviral and neoplastic diseases. Alternatively, the active fraction can be produced by ethyl acetate or similar fractionation of shark blood.

In a first aspect of the present invention there is provided a method for preparing shark serum concentrate useful for inhibiting disease in a mammal, comprising:

(a) adding ethanol to shark blood in an amount sufficient to cause separation of red cells and serum, thereby forming an ethanol-shark blood mixture;

(b) warming the ethanol-shark blood mixture to a temperature and for a time sufficient to evaporate the ethanol but not sufficient to inactivate serum complement; and

c) separating the mixture into precipitate and shark serum concentrate; and.

d) repeating steps (a)-(c) with the shark serum concentrate at least 3 times.

In a second aspect of the present invention there is provided a method for preparing purified shark immunoglobulin useful for inhibiting disease in a mammal, comprising:

a) adding ethanol to shark blood in an amount sufficient to cause separation of red cells and serum at a temperature of about 10°C, thereby forming an ethanol-shark blood mixture;

b) separating the mixture into precipitate and a supernatant comprising shark serum concentrate;

c) adding sufficient ethanol the supernatant of step (b) to achieve a final concentration of about 60% ethanol to form a precipitate;

d) separating the precipitate of step (c);

e) adding a sufficient volume of NaCl to solubilize the precipitate from step (d) and to form a precipitate comprising shark serum immunoglobulin;

f) separating the precipitate from step (e) from the solution;

g) adding a sufficient volume of ethanol to the filtrate from step (f) to reach a final concentraton of about 40% ethanol to form a precipitate and a supernatant;

h) adding a sufficient amount of NaCl to the precipitate from step (g) to solubilize the precipitate, adjusting the pH of the solution to about 7.4 and adding a sufficient volume of ethanol to achieve a final concentration of about 25% ethanol to forming a precipitate comprising shark immunoglobulin;

i) separating the precipitate comprising sharl immunoglobulin from step (h) from the solution.

In a third aspect of the present invention there is provided a method for preparing a purified shark immunoglobulin useful for inhibiting disease in mammals comprising:

a) adding to a volume of shark blood an equal volume of distilled water and ethyl acetate;

b) allowing the ethyl acetate phase to separate from the water phase;

c) separating the ethyl acetate phase from the water phase;

d) adding the volume of ethyl acetate from step (a) and mixing with the water phase of step (c);

e) allowing the ethyl acetate phase to separate from the water phase;

f) separating the ethyl acetate phase from the water phase;

g) repeating steps (d) through (f);

h) removing the water from the ethyl acetate phases;

i) adding acetone to the ethyl acetate phase from step (h) to precipitate the shark immunoglobulin; and

j) separating the purified shark immunoglobulin from the ethylfucetate phase.

In a fourth aspect of the present invention there is provided a composition obtainable by any one of the methods of the present invention for inhibiting Rauscher murine leukaemia virus proliferation or tumour cell proliferation in a mammal.

The present invention provides, in one embodiment, methods for preparing shark serum concentrate that are useful in inhibiting a retroviral and neoplastic disease in a susceptible mammalian cell. Generally, the shark serum is produced by an admixture of ethanol and shark blood, followed by removal of the ethanol, typically by heating to a temperature and for a time sufficient to evaporate the ethanol but not sufficient to inactivate serum proteins such as immunoglobulin and complement. The serum is then separated from any precipitate and is used directly or to form compositions, including pharmaceutical compositions, useful for inhibiting retroviral and neoplastic diseases. Alternatively, the active fraction can be produced by ethyl acetate or similar fractionation of shark blood.

Thus, in one method for preparing serum extracts, ethanol is added to shark blood in an amount sufficient to cause separation of red cells and serum, thereby forming an ethanol-shark blood mixture. Typically the shark blood is obtained frozen and is thawed to, e.g., room temperature, before adding the ethanol. Ethanol may be added in an amount of about 20-35% vol/vol, more usually about 25% vol/vol. The ethanol-shark blood mixture is warmed to a temperature, e.g., to about 21°C-30°C (70-85°F), and for a time sufficient to evaporate the ethanol but typically not sufficient to inactivate any serum complement. The mixture is then separated by centrifugation, filtration or the like into precipitate and shark serum concentrate. The process can then be repeated up to three or more times with the serum to achieve further clarify and concentration of immunoglobulins, including IgM and/or any IgG-like molecules.

In another aspect the invention provides a composition, including pharmaceutical composition, which comprises shark serum produced by a process of admixture of ethanol and shark blood, followed by removal of ethanol, typically by heating but not inactivating serum complement, and separation of serum from any resulting particulate. Alternatively the composition is produced by ethyl acetate extraction. The composition can be formulated in amounts sufficient to inhibit retroviral replication in susceptible cells, or in amounts suitable for treating neoplastic disease, and further comprises a pharmaceutically acceptable carrier or stabilizer, such as maltose or the like. Compositions of shark serum IgM and/or IgG-like molecules can be combined wit:h shark marrow preparations for a synergistic anti-retroviral action. The shark serum composition and immunoglobulins therein can also be lyophilized.

In other embodiments the invention provides compositions for inhibiting a retroviral infection of susceptible mammalian cells. An amount of shark serum, purified IgM and/or IgG-lice molecule, or even whole blood sufficient to inhibit or prevent said retroviral infection, is to be administered to and thereby contacted with the infected or infection-susceptible cells. The retrovirus susceptible to inhibition include the mouse Rauscher Leukemia Virus (RLV). The shark serum concentrate, purified IgM and/or IgG-like fractions, separate or combined with marrow preparations, are to be administered in a variety of ways, including intravenously, topically, intramuscularly or orally.

In another aspect the invention discloses compositions for inhibiting tumor cells. Shark serum concentrate as described herein is to be administered to the tumor cells in an amount sufficient to inhibit growth of the tumor. The tumor cells being inhibited can be in a culture, e.g., in vitro, in an afflicted mammal, or removed from the mammal for ex vivo treatment. The tumors susceptible to inhibition include a variety of sarcomas and leukemias, and further include those which are induced by a retroviral gene.

Description of the Specific Embodiments

The present invention discloses compositions useful for the inhibition or reversion of retroviral-mediated disease in an infected mammal. It has been discovered as part of the present invention that preparations of shark serum and immunoglobulins purified therefrom, IgM and/or IgG-like molecules, inhibit manifestations of retroviral disease in an infected mammal. The shark serum and immunoglobulin preparations are able to inhibit retroviral titer and associated symptoms and either restore cellular functions or prevent their further deterioration. In other aspects of the invention the shark serum preparations and immunoglobulins are used to inhibit development of neoplastic disease, such as sarcomas or lymphomas, in afflicted mammals.

As part of the present invention it has been demonstrated that shark blood, serum and immunoglobulin-containing fractions can be prepared which possess significant anti-retroviral and anti-neoplastic activity. The shark serum concentrate and immunoglobulin fraction(s), when administered to animals infected with the Rauscher murine leukemia virus, inhibit or prevent the development of splenomegaly in the animals in a dose dependent manner, whereas untreated animals develop severe splenomegaly. The Rauscher leukemia virus is a retrovirus widely used as an in vivo model of HIV retrovirus infection. A standard measure of drug effectiveness in the Rauscher model has been the ability to inhibit splenomegaly in infected animals. In the experiments described herein the shark serum concentrate, shark blood, serum immunoglobulin fractions, and ethyl acetate fractions of shark blood substantially and in some cases completely prevent the splenomegaly observed in Rauscher infected control animals. These findings indicate that symptoms of a retroviral disease, can be inhibited or completely prevented, thereby allowing the immune system of the infected individual to respond more appropriately to other antigens for which an individual's response had been severely depressed, thus extending the life of the individual.

The preparations of shark serum concentrate described herein can be used to treat pathological conditions associated with the retroviral infection at the cellular level, such as RLV-induced neurological damage, retroviral-induced neoplastic diseases, programmed cell death, and the like. Retroviruses which can be treated by the serum compositions include Rauscher murine leukemia virus. A disease condition amenable to treatment or inhibition by the shark serum or immunoglobulin (IgM and/or IgG-like molecule) and fraction(s) thereof are identified using, e.g., mammalian cells or animals suspected of undergoing the disease state, e.g., sarcoma cells, which are confirmed to be susceptible to the process, e.g., tumorigenic in the case of the sarcoma cells. To determine that the shark serum or other preparation inhibits the disease process, the cells are treated with the shark serum or specific immunoglobulin preparation and the results compared to an untreated cell sample. In affected cells which have been treated and which demonstrate a inhibition or reversal of a conveniently monitored functional attribute(s), such as neoplastic proliferative capability, the process is determined to be susceptible to treatment according to the present invention. The present methods can also be used in treating autoimmune or autoimmune-associated diseases, particularly those which are associated with immunodeficiencies, as may be associated with RLV infection or the like.

In one embodiment a shark serum preparation of the present invention is prepared by careful separation of blood components such as hemoglobin and cells from serum components. In a preferred embodiment herein, the blood is initially obtained frozen from a supplier. Shark blood is harvested by a variety of different means. As shark blood begins to clot immediately after sacrifice, bleeding must begin as soon as possible. In one method the shark's head is raised and the tail removed and blood allowed to drain into a suitable container. Blood can also be obtained without sacrificing the shark, such as by employing venipuncture of an anesthetized or immobilized shark, but smaller quantities are obtained. Preferably, the harvested blood is promptly frozen to prevent deterioration. Blood may be used from a number of different sharks, such as the Mako, Thresher, Black Tip, or Common Reef Shark, but other species also suffice, e.g., the Sandbar or Nurse sharks. Titers of anti-retroviral and anti-tumor serum activity can be determined by in vitro assays as described herein for a particular species or source. Serum or isolated and purified immunoglobulins from different species can be pooled, if desired, to achieve a particular level of activity. Different immunoglobulins can be combined, e.g., IgM and/or IgG-like molecules, and various combinations with shark marrow can also be prepared for effective use in the therapeutic methods described herein.

To separate the serum from the cellular components the frozen blood is allowed to thaw, typically at room temperature or the like. In a preferred separation and concentration procedure, the thawed blood is admixed with ethanol or suitable alternatives, e.g., fluorinated derivatives of ethanol, at a ratio of about 15-60% ethanol to blood (volume/volume), more preferably about 20-35% ethanol, and most preferably in the example described herein a 25% ethanol to blood mixture is used. Pure ethanol (100%) is preferably used in this separation process. The mixture is gradually brought to a temperature of about 21°C-32°C (70-90°F) with gentle mixing, more preferably about 21°C-30°C (70-85°F), and most preferably in a range of about 21°C-24°C (70-75°F). A heating plate or similar device can be used for this purpose. The mixing should be sufficient to effect a thorough mixture of the blood and alcohol and simultaneously avoiding scalding the preparation at the heating surface. The gentle mixing of the warmed preparation continues until the alcohol has been substantially evaporated from the mixture, generally about 1 to 1½ hours for a 100 ml preparation. The resulting preparation typically will contain a precipitate, and this fraction is separated from the fluid (serum concentrate), typically by centrifugation, filtration or the like. The concentration process can optionally be repeated with the serum concentrate, i.e., repeating the ethanol treatment, heating and mixing, and separating, until the resulting serum concentrate is substantially clear and the precipitate is substantially eliminated at the final separation step. Typically the process is repeated three to four times to reach the end product. A subsequent test of the final preparation is performed by mixing a sample thereof with ethanol, heatinc and mixing; the absence of further precipitate indicates a substantially pure serum product.

For preparing a pharmaceutically acceptable composition, the shark serum concentrate and fractions obtained therefrom, e.g., purified IgM and/or IgG-like molecules, will typically be sterilized in a manner well known to those familiar with preparing pharmaceutically acceptable serum preparations, e.g., by filtration, irradiation, etc. Methods for preparing pharmaceutically acceptable serum preparations are described in, e.g., U.S. Patent Nos. 4,587,121, 3,903,262 and 4,186,192.

The shark serum or immunoglobulin compositions may be administered to persons or mammals suffering from, or predisposed to suffer from retroviral-associated disease or cancer. The shark serum and immunoglobulin thereof is believed to restore functionality, such as immunoprolif erative capacity. Thus, not only is replication or spread of the virus impeded by the treatment, but the mammal regains, or retains, a responsive immune system and therefore is able to respond to other antigenic challenges.

The compositions also find use for pre- or post-exposure prophylaxis, following dirty needle injuries to health care workers or routinely accompanying blood transfusions or to persons in danger of becoming exposed to infected body or culture fluids. For post-exposure prophylaxis, administration is begun shortly after the suspected inoculation and continues for at least about two to four weeks thereafter, followed by additional dosages or long term maintenance dosages as may be necessary to inhibit growth of the virus and disease and/or to maintain immunity thereto.

EP 0 835 124 B1

The pharmaceutical serum and immunoglobulin compositions are intended for parenteral, topical, oral, or local administration for prophylactic and/or therapeutic treatment. Preferably, the pharmaceutical compositions are administered orally or parenterally, i.e., intravenously, intraperitoneally, subcutaneously, or intramuscularly. Thus, this invention provides compositions for oral, topical or parenteral administration which comprise a solution of a shark serum concentrate, substantially purified immunoglobulin, and/or shark marrow in a pharmaceutically acceptable carrier, preferably an aqueous carrier. A variety of aqueous carriers may be used, e.g., water, buffered water, 0.4% saline, 0.3% glycine, and the like, and may include other proteins for enhanced stability, such as albumin, lipoprotein, globulin, etc., subjected to mild chemical modifications or the like. The compositions of the invention can also be formulated into a cream or salve for topical or transdermal administration, e.g., at 5-25% concentration. The compositions may be sterilized by conventional, well known sterilization techniques. The resulting solutions may be packaged for use or filtered under aseptic conditions and lyophilized, the lyophilized preparation being combined with a sterile solution prior to administration. The compositions may contain pharmaceutically acceptable auxiliary substances as required to approximate physiological conditions, such as a pH adjusting and buffering agents, tonicity adjusting agents and the like, for example, sodium acetate, sodium lactate, sodium chloride, potassium chloride, calcium chloride, stabilizers (e.g., maltose (1-20%), etc.

The concentration of the shark serum, immunoglobulin preparation and/or marrow in these pharmaceutical formulations can vary widely, i.e., from less than about 10%, usually at or at least about 25% to as much as 75 or 90% by weight and will be selected primarily by fluid volumes, viscosities, etc., in accordance with the particular mode of administration selected and the disease being treated. Actual methods for preparing orally, topically and parenterally administrable compositions will be known or apparent to those skilled in the art and are described in detail in, for example, Remington's Pharmaceutical Science, 17th ed., Mack Publishing Company, Easton, PA (1985).

Determination of an effective amount of a composition of the invention to inhibit retroviral-mediated disease or cancer in a patient can be determined through standard empirical methods which are well known in the art. For example, reversal of impairment of immune function, e.g., restoration of lymphoproliferative response to recall antigen (e.g., influenza), alloantigens or mitogens such as PWM or PHA, and thus efficacy of the subject compositions, can be monitored with a variety of well known in vitro T-cell proliferative response procedures.

Compositions of the invention are to be administered to a host already suffering from a retroviral infection or neoplasm, as described above, in an amount sufficient to prevent or at least partially arrest the development of the ensuing immunodeficiency disease and its complications, or the susceptible tumor, as more fully described below. An amount adequate to accomplish this is defined as a "therapeutically effective dose." Amounts effective for this use will vary considerably and depend on the severity of the infection or disease and the weight and general state of the patient being treated, but generally range from about 5 mg/kg to about 1,000 mg/kg host body weight of shark serum concentrate, immunoglobulin and/or marrow per application, with dosages of from about 10 mg/kg to about 200 mg/kg per application being more commonly used. Administration is daily, weekly or less frequently, as necessary depending on the response to the disease and the patient's tolerance of the therapy. Maintenance dosages over a prolonged period of time may be needed, and dosages may be adjusted as necessary. The period of administration will generally be sufficient to restore the immune system of the host, such that effective immune responses can be mounted against a variety of antigens, most desirably the RLV virus in the case of mammals infected with RLV, or to eliminate cr substantially inhibit the growth of the cancer cells. If an individual's restored immune system is not able to eliminate the disease, maintenance dosages over a prolonged period may be necessary. Also, it must be kept in mind that the materials of the present invention may be employed in life-threatening or potentially life threatening situations. In such cases, it is possible and may be felt desirable by the treating physician to administer substantial excesses of these compositions. For veterinary uses higher levels may be administered as necessary.

In prophylactic applications, compositions of the present invention are to be administered to a mammal susceptible to or otherwise at risk of retroviral-mediated disease to enhance the patient's own immunologic capabilities. Such an amount is defined to be a "prophylactically effective dose." In this use, the precise amounts again depend on the patient's state of health and weight, but generally range from about 1 mg/kg to about 250 mg/kg body weight, more commonly from about 10 mg/kg to about 100 mg/kg of body weight.

Single or multiple administrations of the compositions are carried out with the dose levels and pattern being selected by the treating physician. In any event, the pharmaceutical formulations of the shark serum concentrate, purified immunoglobulin preparation, and/or marrow should provide a quantity of the inhibitor sufficient to effectively inhibit the retroviral-mediated disease or tumor in the treated host.

The compositions of the invention can also be employed for ex vivo therapy. By ex vivo or extracorporeal therapy is meant that therapeutic manipulations are performed on host cells and fluids outside the body. For example, lymphocytes or other target cells may be removed from a patient and treated with high doses of the composition, providing a concentration of inhibitor to the cell far in excess of levels which could be accomplished or tolerated by a patient. Following treatment, the cells are returned to the host to treat the disease.

A composition of the invention can be combined with one or more other pharmaceutical compositions for a variety of therapeutic uses, e.g., enhanced therapeutic activity against RLV retroviruses, or cancer. For example, in the treatment of RLV infection, the pharmaceutical compositions of the present invention may be administered alone or as adjunct therapy, and shark serum concentrate. The IgM fraction can also be combined with the IgG-like fraction to achieve enhanced efficacy. Serum and/or purified immunoglobulins can also be combined with shark marrow preparations. When administered as adjunct therapy, the preparations can be administered in conjunction with the other treatment modalities, or separately at different intervals.

The compositions of the present invention also find use in vitro. The compositions can be used to inhibit retroviral induced death of cultured cells, such as certain hybridoma or other lymphocyte lines which are susceptible to retroviral infection. The preparations of the invention can also be used in screening assays to assess effective levels of anti-retroviral compounds or other treatments. In addition, by determining whether a retrovirus-mediated dysfunction or death of a patient's cells is susceptible to inhibition or reversal by a composition of the invention, appropriate therapy can be more readily instituted or, alternatively, the effect of other treatment modalities, such as other anti-RLV regimens, can be determined. Thus, a diagnostic for assessing the efficacy of, e.g., anti-RLV therapy may also be provided. Detecting changes in vitro regarding the level of HIV susceptibility, or restoration of immune function, e.g., response to recall antigens, to alloantigens, or to mitogens such as PWM or PHA, provides an indication of in vivo activity of the shark serum or immunoglobulin compositions intended for treatment in accordance with the present invention.

To monitor changes in the level of immune function in a cell population, control values of immune function may be determined from cells from the general population or from the patient prior to commencement of therapy. Since immune function may vary considerably among patients, determination of each patient's pre-treatment immune function is preferred. The level of immune function in cells, e.g., lymphocytes in the case of RLV-infected mammals, is then monitored during therapy. This level is compared to the level of the immune function in cells not exposed to therapy, and effectiveness of therapy is assessed by an increased level in the measured. immune function during or post-therapy.

The shark serum concentrate and purified IgM and/or IgG-like immunoglobulin of the present invention can also be used as an anti-neoplastic agent. Among the neoplastic diseases inhibited by the serum concentrate are sarcomas, leukemias, and carcinomas, including those which may be induced by a retroviral gene. Determination of an effective amount of shark serum concentrate of the invention sufficient to inhibit growth of the neoplastic cells may be determined by, for example, monitoring metastatic sites with a variety of procedures, e.g., in vivo imaging or ex vivo diagnostic techniques. Other cancer markers may also be used to monitor therapy with the serum concentrates of the invention, e.g., the CEA assay, or the PSA assay for prostate carcinoma, which assay is commercially available.

The therapeutic compositions of the invention are to be administered to a patient already suffering from a neoplasm, e.g., sarcoma, leukemia or carcinoma, in an amount sufficient to cure or at least partially arrest the disease. An amount adequate to accomplish this is defined as "therapeutically effective dose." Amounts effective for this use will depend on the severity of the neoplasm and its location, particularly when a metastatic site is implicated, and the weight and general state of the patient being treated, but generally range from about 10 mg/kg to about 1,000 mg/kg host body weight of serum concentrate and/or immunoglobulin per day, with dosages of from about 25 mg/kg to about 125 mg/kg per day being more commonly used. Maintenance dosages over a prolonged period of time may be adjusted as necessary. As the shark serum concentrates and immunoglobulins may be employed in advanced disease states, that is, life-threatening or potentially life threatening neoplastic disease, it is possible and may be felt desirable by the treating physician to administer substantial excesses of these compositions.

Single or multiple administrations of the compositions can be carried out with the dose levels and pattern being selected by the treating physician. In any event, the pharmaceutical formulations should provide a quantity of composition of the invention sufficient to effectively inhibit the neoplastic disease. The pharmaceutical compositions of the present invention may be administered alone or as adjunct therapy. The compositions may be administered with, e.g., taxol, cis-platin, tamoxifen, etoposide phosphate, doxorubicin, daunomycin, endocrine therapy, etc. When administered as adjunct therapy, the compositions of the present invention may be administered in conjunction with the other treatment modalities, or separately at different intervals.

As with compositions described herein for inhibiting retroviral-mediated disease, the shark serum and marrow preparations and immunoglobulin of the invention can also be used in ex vivo therapy of neoplastic disease. For example, bone marrow or other target cells or tissues are removed from a patient and treated with high doses of compositions which comprise the composition of the invention, which optionally can be conjugated to cytotoxic agents such as toxins, drugs, labels, etc., proving a therapeutic concentration of the compositions far in excess of levels which could be accomplished or tolerated by the patient. Following treatment to eliminate the neoplastic cells in the target cell population or tissue, the cells or tissues are return to the patient.

The following experimental examples are offered by way of illustration, not by limitation.

EXAMPLE I

This Example describes the preparation of serum concentrates from shark blood.

Whole shark blood from Mako shark was obtained frozen. Blood was obtained from the sharks by severing the tail and elevating the body, allowing the blood to drain into a container which was sealed and frozen in a refrigerated compartment. The blood was kept frozen until delivered to the laboratory. The frozen blood was allowed to thaw at room temperature.

In a typical preparation, 100 ml of the thawed shark blood was treated with 25% (by volume) pure ethanol at room temperature. The mixture is then brought to a temperature of about 21°C-24°C (70-75°F), with mixing sufficient to accomplish a thorough mixture of the blood and alcohol and so as to prevent scalding the preparation at the heating surface. The gentle mixing of the warmed preparation continues until the alcohol has been substantially evaporated from the mixture, generally about 1 to 1½ hours for a 100 ml preparation. The resulting preparation contained a precipitate, and this was removed by centrifugation (Physician's Compact Centrifuge, Clay Adams, Inc.) at 1,315 x g for 40-45 min. and the precipitate discarded. This process of ethanol treatment, heating and mixing, centrifugation and decanting was repeated three to four times. The resulting fluid is very clear and only a trace of precipitate is obtained at the final centrifugation step. A subsequent test of the final preparation by mixing with ethanol, heating and mixing followed by centrifugation indicates a substantially pure product with no precipitate in the centrifuging vessel.

The original shark blood had a hemoglobin concentration of 16g per 100 ml and a total protein content of 6.6g per 100 ml. The shark blood concentrate used in the Rauscher virus inhibition examples described below had a hemoglobin content of 4.6g per 100 ml and a protein content of 1.5g per 100 ml. Testing two preparations of serum concentrate with reagents specific for human IgM indicated concentrations of approximately 368 mg/dl for the first and 155 mg/dl for the second, and a reagent specific for human IgG indicated concentrations of approximately 139 mg/dl for the first and 52 mg/dl for the second.

EXAMPLE II

This Example describes the use of the serum preparation described in Example I to inhibit manifestations of retrovirus infection in a mammal.

Efficacy of the shark serum preparation as an anti-retroviral agent was determined using the Rauscher virus disease model. Rauscher is a pathogenic murine retrovirus in mice, typically causing erythroid colonies in the spleen of mice leading to a severe splenomegaly, and also causes a erythroleukemia. In this study, BALB/c mice treated with the shark serum preparation and untreated controls were infected with virus preparations.

BALB/c mice weighing 12-15 grams each were used as test animals. The mice were obtained from Harlan Sprague Dawley, Indianapolis, IN, and were quarantined for a minimum of five days before they were used for testing. The mice were held at stable temperature and observed daily for signs of disease, stress, injury, and external parasites.

The test was conducted as follows. The animals were divided into five groups. Group I, which consisted of twelve animals, served as a control, in that mice were not challenged with Rauscher virus, but were injected IP with 0.2 ml of physiological saline solution daily for five days. Twelve animals of Group 2 were challenged with the Rauscher virus on Day 0 of the study, and received injections IP of physiologic saline solution daily for five days. Group 3A consisted of six animals that were challenged with Rauscher virus on Day 0 and received 0.5 ml shark serum preparation by intraperitoneal injection every day for five days. Group 3B consisted of six animals that were challenged with Rauscher virus on Day 0 and received 0.25 ml shark serum preparation by intraperitoneal injection every day for five days. Group 3C consisted of six animals that were challenged with Rauscher virus on Day 0 and received 0.1 ml shark serum preparation by intraperitoneal injection every day for five days. Each mouse was injected intraperitoneally with 0.1 ml Rauscher leukemia virus infected cells (06-000-000; Advanced Biotechnologies, Inc., MD). Immediately following virus injection on Day 0, the animals received the first injection of saline or the shark serum preparation. Animals were observed daily for 21 days, and the experiment was terminated on day 21 with the weights of all survivors recorded prior to sacrifice. Spleens were removed from all animals immediately following sacrifice, weighed and recorded.

The results, which can be seen in Table 1, showed that Group 1 animals which received only saline had a mean spleen weight per mouse of 120 mg. Group 2 animals that were challenged with Rauscher virus but received only saline treatment had massive spleens, with a mean spleen weight per mouse of 3,800 mg. These control groups demonstrate the establishment of the retroviral infection and the advancing disease in infected animals. In contrast to the saline treated animals, animals which received 0.5 ml, 0.25 ml, or 0.1 ml of shark serum preparations had dramatically smaller spleens, with mean spleen weights per mouse of 110 mg, 398 mg, and 872 mg, respectively.

TABLE 1

| Group | Mean Final Body Wt. Per Mouse (g) | Mean Spleen Weight Per Mouse (mg) |
|---|---|---|
| I (neg. ctrl) | 23.0 | 120 |

TABLE 1   (continued)

| Group | Mean Final Body Wt. Per Mouse (g) | Mean Spleen Weight Per Mouse (mg) |
|---|---|---|
| II (pos. ctrl) | 21.5 | 3800 |
| III-A | 22.4 | 110 |
| III-B | 27.3 | 398 |
| III-C | 22.4 | 872 |

The results confirm that the shark serum preparation contained significant anti-retroviral activity and effectively prevented, in the case of the .5 ml treatment, or inhibited in the case of the lower doses, the normal progression of retroviral disease in the infected animals.

EXAMPLE III

This Example describes the use of fractionated shark serum preparations to inhibit manifestations of retrovirus infection in mice using the Rauscher virus disease model.

Whole shark blood was obtained as described in Example I. The blood serum was prepared and fractionated for testing in the Rauscher test, as follows. To 100 ml of blood was added 800 ml of distilled water and 300 ml of ethanol, followed by mixing for 10 min at 10°C and centrifugation at 1600 x g for 10 min at 10°C. The precipitate (designated "Ppt. I") was saved for testing (as a resuspension in phosphate buffered saline, PBS), and the supernatant (designated "Sup. I"), 750 ml., was combined with ethanol to achieve a final concentration of 60% ethanol, forming a precipitate. The mixture was centrifuged at 22,000 x g for 20 min at 4°C, the supernatant therefrom designated "Sup. II." The precipitate, "Ppt. II," was combined with 30 mM NaCl (750 ml) and filtered. The resulting precipitate ("Ppt. III) was discarded and the filtrate treated with ethanol to a final concentration of 30% ethanol. This was centrifuged at 22,000 x g for 20 min at 4°C and the supernatant ("Sup. III") saved. The precipitate ("Ppt. IV") was treated with 30 mM NaCl (100 ml) (a portion of Ppt. IV was also resuspended in PBS for testing), the pH adjusted to 7.4 with 0.5 M $Na_2HPO_4$, and then mixed with ethanol to achieve a final concentration of 25% ethanol. The solution was centrifuged at 22,000 x g for 20 min at 4°C, and the precipitate ("Ppt. V"), comprising a purified immunoglobulin fraction including an IgG-like molecule, was harvested.

Separately, a preparation of shark blood was extracted with ethyl acetate. To 500 ml of blood was added 500 ml of $dH_2O$, and three extractions with 500 ml portions of ethyl acetate were performed. The resulting 1400 ml of ethyl acetate was dried over anhydrous sodium carbonate and then evaporated to a 50 ml volume. A precipitate was then formed upon the addition of 300 ml acetone. The precipitate was centrifuged and air dried. Approximately 2 grams of a tan to off-white powder resulted and is referred to as the ethyl acetate fraction. About 0.5 grams of the powder was resuspended in 50 ml PBS for testing.

BALB/c mice weighing 12-15 grams each were used as test animals. The mice were obtained from Simonsen Laboratories, Gilroy, CA, and were quarantined for a minimum of five days before they were used for testing. The mice were held at stable temperature and observed daily as described above.

The tests were conducted as follows. For challenge virus, Rauscher leukemia virus infected cells (06-000-000) were obtained from Advanced Biotechnologies, Inc., MD), and the virus passed from passage mice. For the challenge procedure, all groups scheduled to be challenged with the Rauscher virus were challenged on Day 0, and each mouse was injected intraperitoneally (IP) with 0.25 ml virus. For the treatment procedure, each mouse scheduled to be treated was injected IP with 0.5 ml of shark blood or the specified fraction for a duration of five days, from Day 1 through Day 5. Mortality counts were recorded daily for all groups. The study was terminated on day 28. Spleens were removed from animals immediately following sacrifice and weighed.

The results, which can be seen in Table 2, showed that Group 1 animals which received only saline had a mean spleen weight per mouse of 98 mg. Group 2 animals that were challenged with Rauscher virus but received only saline treatment had a mean spleen weight per mouse of 3,620 mg. These control groups demonstrate the establishment of the retroviral infection and the advancing disease in infected animals.

In contrast to the saline treated animals, animals which received 0.5 ml of whole blood (Group 3), Ppt. V (Group 11) or ethyl acetate concentrate (Group 12) had dramatically smaller spleens, with mean spleen weights per mouse of 226 mg, 148 mg, and 96 mg, respectively.

TABLE 2

| Group | No. Mice | Test | Rauscher Virus | Mean Spleen Weight per mouse (mg) |
|---|---|---|---|---|
| 1 | 10 | saline | no | 98 |
| 2 | 10 | saline | yes | 3620 |
| 3 | 5 | blood | yes | 226 |
| 4 | 5 | Ppt. I | yes | 2980 |
| 5 | 5 | Sup. I | yes | 2000 |
| 6 | 5 | Ppt. II | yes | 2200 |
| 7 | 5 | Sup. II | yes | 3460 |
| 8 | 5 | Ppt. IV | yes | 1880 |
| 9 | 5 | Sup. III | yes | 3540 |
| 10 | 5 | Ppt. V | yes | 148 |
| 11 | 5 | Sup. IV | yes | 3700 |
| 12 | 5 | Ethyl Acetate | yes | 96 |

The results confirm that the shark blood and serum immunoglobulin preparations, including an IgG-like fraction, contained significant anti-retroviral activity and effectively prevented or inhibited the progression of retroviral disease in the spleen of infected animals.

A separate study was performed with a different preparation of purified shark immunoglobulin in the Rauscher model and a fraction ("Fr. 2") obtained during the purification process. The study was performed essentially as described above, summarized below:

TABLE 3

| Group | No. Mice | Test[a] | Rauscher Virus | Mean Spleen Weight per mouse (mg) |
|---|---|---|---|---|
| 1 | 8 | saline | no | 103.1 |
| 2 | 8 | saline | yes | 3532.5 |
| 3 | 8 | 10 mg/kg Ig | yes | 116.1 |
| 4 | 8 | 5 mg/kg Ig | yes | 154.4 |
| 5 | 8 | 1 mg/kg Ig | yes | 273.9 |
| 6 | 8 | 1 mg/kg Fr.2 | yes | 104.7 |
| 7 | 8 | .5 mg/kg Fr.2 | yes | 105.2 |

[a] = Test substance administered daily (IP) for five days; all animals sacrificed on day 25 of the study.

This study confirms the anti-retroviral efficacy of the shark immunoglobulin-containing fraction that was observed in the study reported in Table 2.

EXAMPLE IV

This Example demonstrates a significant anti-tumor activity of the shark serum preparation described in Example I.

The tumor used in this study was Sarcoma 180 obtained from the American Type Culture Collection and passed at weekly intervals as an ascites in non-treated female Swiss-Webster mice. To prepare the inoculum of tumor cells for the present study, ascites fluid from a mouse with a 7-10 day ascites was aspirated with sterile technique. The tumor cells were checked for viability using trypan-blue staining. A tumor cell count was then determined, and cells diluted with normal saline or phosphate buffered saline to obtain a final concentration of $1-2 \times 10^6$ cells/mm$^3$. This

suspension is used for injection in mice. The final dilution is plated onto trypticase soy agar to determine that it is free from bacterial contamination.

For injection into animals, 0.1 ml of tumor cell suspension is inoculated into the left hind leg muscle of each mouse. Inoculated mice were then placed into a large cage and then randomly segregated into groups of ten mice and then housed under routine conditions. Mice were weighed on the day of inoculation, day 7 and day 14 at the time of sacrifice.

Treatment of the mice was usually initiated one day after injection of the tumor and was continued daily for five days. In one group the treatment was not initiated until day 7, at which time the developing tumor was palpable. In this instance the treatment continued for 5-7 days prior to sacrifice. At the end of the 14 day observation period the mice were sacrificed by cervical dislocation or ether anesthesia. The skin over the left hind leg was removed to expose the tumor, and the leg with tumor was removed at the hip joint. Any residual skin was removed and the leg/tumor was weighed. The mean value of the normal leg was subtracted from the weight of the leg with the tumor to provide an estimate of the actual tumor weight. Percent tumor inhibition was determined as follows:

$$\% \text{ Inhibition} = \frac{\text{Mean tumor weight (test)}}{\text{Mean tumor weight (control)}} \times 100$$

There were ten mice per group. The test mice were treated on day one (the day after the tumor implant) and continued for five days. Tumor weights were determined on day 14. Ten mice served as controls and were treated with 0.5 ml saline, ten mice received 0.25 ml shark serum preparation, and ten mice received 0.5 ml shark serum preparation. All treatments were administered by intraperitoneal injection.

The results, shown in Table 4, indicated that the shark serum preparation contained significant anti-tumor activity, with mean tumor inhibition of 57.7% and 98.4% for the .25 ml and .5 ml shark serum preparation treatments, respectively, when compared to tumors of the saline treated control animals.

TABLE 4

| Group | Mean Tumor Wt. (g) | % Inhibition |
|---|---|---|
| Control | 2.43 g. | -- |
| 0.25 ml Serum Conc. | 1.03 g. | 57.7% |
| 0.5 ml Serum Conc. | 0.04 g. | 98.4% |

**Claims**

1. A method for preparing shark serum concentrate useful for inhibiting disease in a mammal, comprising:

(a) adding ethanol to shark blood in an amount sufficient to cause separation of red cells and serum, thereby forming an ethanol-shark blood mixture;

(b) warming the ethanol-shark blood mixture to a temperature and for a time sufficient to evaporate the ethanol but not sufficient to inactivate serum complement; and

c) separating the mixture into precipitate and shark serum concentrate; and.

d) repeating steps (a)-(c) with the shark serum concentrate at least 3 times.

2. The method of claim, wherein the shark blood is obtained frozen and is thawed before adding the ethanol of step (a).

3. The method of claim 1 or 2, wherein the ethanol of step (a) is added to shark blood in an amount of 20% to 35% vol/vol.

4. The method of any preceding claim wherein the ethanol-shark blood mixture of step (b) is warmed to a temperature of about 21°C to about 30°C while mixing.

5. The method of claim 4, wherein the ethanol-shark blood mixture is maintained at said temperature while mixing for at least about one hour.

6. The method of any preceding claim, wherein the separation of step (c) is accomplished by centrifugation or filtration.

7. A method for preparing purified shark immunoglobulin useful for inhibiting disease in a mammal, comprising:

   a) adding ethanol to shark blood in an amount sufficient to cause separation of red cells and serum at a temperature of about 10°C, thereby forming an ethanol-shark blood mixture;

   b) separating the mixture into precipitate and a supernatant comprising shark serum concentrate;

   c) adding sufficient ethanol to the supernatant of step (b) to achieve a final concentration of about 60% ethanol to form a precipitate;

   d) separating the precipitate of step (c);

   e) adding a sufficient volume of NaCl to solubilize the precipitate from step (d) and to form a precipitate comprising shark serum immunoglobulin;

   f) separating the precipitate from step (e) from the solution;

   g) adding a sufficient volume of ethanol to the filtrate from step (f) to reach a final concentration of about 40% ethanol to form a precipitate and a supernatant;

   h) adding a sufficient amount of NaCl to the precipitate from step (g) to solubilize the precipitate, adjusting the pH of the solution to about 7.4 and adding a sufficient volume of ethanol to achieve a final concentration of about 25% ethanol to form a precipitate comprising shark immunoglobulin;

   i) separating the precipitate comprising shark immunoglobulin from step (h) from the solution.

8. A method for preparing a purified shark immunoglobulin useful for inhibiting disease in mammals comprising:

   a) adding to a volume of shark blood an equal volume of distilled water and ethyl acetate;

   b) allowing the ethyl acetate phase to separate from the water phase;

   c) separating the ethyl acetate phase from the water phase;

   d) adding the volume of ethyl acetate from step (a) and mixing with the water phase of step (c);

   e) allowing the ethyl acetate phase to separate from the water phase;

   f) separating the ethyl acetate phase from the water phase;

   g) repeating steps (d) through (f);

   h) removing the water from the ethyl acetate phases;

   i) adding acetone to the ethyl acetate phase from step (h) to precipitate the shark immunoglobulin; and

   j) separating the purified shark immunoglvbulin from the ethylfucetate phase.

9. A composition obtainable by the method of any one of the preceding claims for use in inhibiting Rauscher mutine leukaemia virus proliferation or tumour cell proliferation in a mammal.

10. The composition of Claim 9, wherein the composition is to be administered intravenously or intramuscularly.

11. The composition of claim 9 or 10, wherein the tumour is a sarcoma or leukaemia.

**12.** The composition of any one of claims 9 to 11, further comprising a pharmaceutically acceptable carrier or stabiliser.

**13.** The composition of claim 12, wherein the pharmaceutically acceptable stabiliser is maltose.

**Patentansprüche**

**1.** Verfahren zur Herstellung von Haiserumkonzentrat, das zur Hemmung von Erkrankungen in einem Säuger nützlich ist, welches umfaßt:

(a) Zugeben von Ethanol zu Haiblut in einer ausreichenden Menge, um die Trennung von roten Blutkörperchen und Serum zu bewirken, wodurch eine Ethanol-Haiblut-Mischung gebildet wird;

(b) Erwärmen der Ethanol-Haiblut-Mischung auf eine Temperatur und für einen Zeitraum, die ausreichend sind, um das Ethanol zu verdampfen, aber nicht ausreichend, um das Serumkomplement zu inaktivieren; und

(c) Trennen der Mischung in Niederschlag und Haiserumkonzentrat; und

(d) wenigstens 3-maliges Wiederholen der Schritte (a) bis (c) mit dem Haiserumkonzentrat.

**2.** Verfahren nach Anspruch 1, **dadurch gekennzeichnet, daß** das Haiblut gefroren erhalten wird und vor dem Zugeben des Ethanols von Schritt (a) aufgetaut wird.

**3.** Verfahren nach Anspruch 1 oder 2, **dadurch gekennzeichnet, daß** das Ethanol von Schritt

(a) zu Haiblut in einer Menge von 20 bis 35 Vol.-% zugegeben wird.

**4.** Verfahren nach einem der vorangehenden Ansprüche, **dadurch gekennzeichnet, daß** die Ethanol-Haiblut-Mischung von Schritt (b) unter Mischen auf eine Temperatur von etwa 21°C bis etwa 30°C erwärmt wird.

**5.** Verfahren nach Anspruch 4, **dadurch gekennzeichnet, daß** die Ethanol-Haiblut-Mischung bei besagter Temperatur unter Mischen für wenigstens etwa eine Stunde gehalten wird.

**6.** Verfahren nach einem der vorangehenden Ansprüche, **dadurch gekennzeichnet, daß** die Trennung von Schritt (c) durch Zentrifugation oder Filtration durchgeführt wird.

**7.** Verfahren zur Herstellung von gereinigtem Hai-Immunglobulin, das zur Hemmung von Erkrankungen in einem Säuger nützlich ist, welches umfaßt:

a) Zugeben von Ethanol zu Haiblut in einer ausreichenden Menge, um die Trennung von roten Blutkörperchen und Serum zu bewirken, bei einer Temperatur von etwa 10°C, wodurch eine Ethanol-Haiblut-Mischung gebildet wird;

b) Trennen der Mischung in Niederschlag und einen Überstand, der Haiserumkonzentrat umfaßt;

c) Zugeben von ausreichend Ethanol zum Überstand von Schritt (b), um eine Endkonzentration von etwa 60 % Ethanol zu erreichen, um einen Niederschlag zu bilden;

d) Abtrennen des Niederschlags von Schritt (c);

e) Zugeben eines ausreichenden Volumens NaCl, um den Niederschlag aus Schritt (d) löslich zu machen und einen Niederschlag zu bilden, der Haiserum-Immunglobulin umfaßt;

f) Abtrennen des Niederschlags aus Schritt (e) von der Lösung;

g) Zugeben eines ausreichenden Volumens Ethanol zum Filtrat aus Schritt (f), um eine Endkonzentration von etwa 40 % Ethanol zu erreichen, um einen Niederschlag und einen Überstand zu bilden;

h) Zugeben einer ausreichenden Menge NaCl zum Niederschlag aus Schritt (g); um den Niederschlag löslich zu machen, Einstellen des pHs der Lösung auf etwa 7,4 und Zugeben eines ausreichenden Volumens Ethanol, um eine Endkonzentration von etwa 25 % Ethanol zu erreichen, um einen Niederschlag zu bilden, der Hai-Immunglobulin umfaßt;

i) Abtrennen des Niederschlags, der Hai-Immunglobulin umfaßt, aus Schritt (h) von der Lösung.

8. Verfahren zur Herstellung eines gereinigten Hai-Immunglobulins, das zur Hemmung von Erkrankungen in Säugern nützlich ist, welches umfaßt:

a) Zugeben eines gleichen Volumens von destilliertem Wasser und Ethylacetat zu einem Volumen Haiblut;

b) Trennenlassen der Ethylacetat-Phase von der Wasser-Phase;

c) Abtrennen der Ethylacetat-Phase von der Wasser-Phase;

d) Zugeben des Volumens von Ethylacetat aus Schritt (a) und Vermischen mit der Wasser-Phase von Schritt (c);

e) Trennenlassen der Ethylacetat-Phase von der Wasser-Phase;

f) Abtrennen der Ethylacetat-Phase von der Wasser-Phase;

g) Wiederholen der Schritte (d) bis (f);

h) Entfernen des Wassers aus den Ethylacetat-Phasen;

i) Zugeben von Aceton zur Ethylacetat-Phase aus Schritt (h), um das Hai-Immunglobulin auszufällen; und

j) Abtrennen des gereinigten Hai-Immunglobulins von der Ethylacetat-Phase.

9. Zusammensetzung, erhältlich mit dem Verfahren nach einem der vorangehenden Ansprüche, zur Verwendung bei der Hemmung von Rauscher-Mäuseleukämie-virus-Proliferation oder Tumorzell-Proliferation in einem Säuger.

10. Zusammensetzung nach Anspruch 9, **dadurch gekennzeichnet, daß** die Zusammensetzung intravenös oder intramuskulär verabreicht werden soll.

11. Zusammensetzung nach Anspruch 9 oder 10, **dadurch gekennzeichnet, daß** der Tumor ein Sarkom oder Leukämie ist.

12. Zusammensetzung nach einem der Ansprüche 9 bis 11, **dadurch gekennzeichnet, daß** sie weiter ein pharmazeutisch annehmbaren Trägerstoff oder Stabilisator umfaßt.

13. Zusammensetzung nach Anspruch 12, **dadurch gekennzeichnet, daß** der pharmazeutisch annehmbare Stabilisator Maltose ist.

**Revendications**

1. Procédé pour la préparation d'un concentré de sérum de requin utile pour inhiber une maladie chez un mammifère, comprenant :

(a) l'addition d'éthanol à du sang de requin en une quantité suffisante pour provoquer la séparation des globules rouges et du sérum, en formant ainsi un mélange éthanol-sang de requin ;
(b) le chauffage du mélange éthanol-sang de requin à une température et pendant un temps suffisants pour l'évaporation de l'éthanol mais insuffisants pour l'inactivation du complément sérique ; et
(c) la séparation du mélange en un précipité et un concentré de sérum de requin ; et
(d) la répétition des étapes (a) à (c) avec le concentré de sérum de requin au moins 3 fois.

**2.** Procédé suivant la revendication 1, dans lequel le sang de requin est obtenu à l'état congelé et est décongelé avant l'addition de l'éthanol de l'étape (a).

**3.** Procédé suivant la revendication 1 ou 2, dans lequel l'éthanol de l'étape (a) est ajouté à du sang de requin en une quantité de 20% à 35% en volume/volume.

**4.** Procédé suivant l'une quelconque des revendications précédentes, dans lequel le mélange éthanol-sang de requin de l'étape (b) est chauffé à une température comprise dans l'intervalle d'environ 21°C à environ 30°C tout en mélangeant.

**5.** Procédé suivant la revendication 4, dans lequel le mélange éthanol-sang de requin est maintenu à ladite température tout en étant mélangé pendant au moins environ une heure.

**6.** Procédé suivant l'une quelconque des revendications précédentes, dans lequel la séparation de l'étape (c) est effectuée par centrifugation ou filtration.

**7.** Procédé pour la préparation d'une immunoglobuline de requin purifiée utile pour inhiber une maladie chez un mammifère, comprenant :

a) l'addition d'éthanol à du sang de requin en une quantité suffisante pour provoquer la séparation des globules rouges et du sérum à une température d'environ 10°C, en formant ainsi un mélange éthanol-sang de requin ;
b) la séparation du mélange en un précipité et un surnageant comprenant le concentré de sérum de requin ;
c) l'addition d'une quantité suffisante d'éthanol au surnageant de l'étape b) pour atteindre une concentration finale d'environ 60% d'éthanol pour former un précipité ;
d) la séparation du précipité de l'étape c) ;
e) l'addition d'un volume suffisant de NaCl pour solubiliser le précipité de l'étape d) et pour former un précipité comprenant une immunoglobuline de sérum de requin ;
f) la séparation du précipité de l'étape e) de la solution ;
g) l'addition d'un volume suffisant d'éthanol au filtrat de l'étape f) pour atteindre une concentration finale d'environ 40% d'éthanol afin de former un précipité et un surnageant ;
h) l'addition d'une quantité suffisante de NaCl au précipité de l'étape g) pour solubiliser le précipité, l'ajustement de pH de la solution à environ 7,4 et l'addition d'un volume suffisant d'éthanol pour atteindre une concentration finale d'environ 25% d'éthanol afin de former un précipité comprenant une immunoglobuline de requin ;
i) la séparation du précipité comprenant l'immunoglobuline de requin de l'étape h) de la solution.

**8.** Procédé pour la préparation d'une immunoglobuline de requin purifiée utile pour inhiber une maladie chez les mammifères, comprenant les étapes consistant :

a) à ajouter à un volume de sang de requin un volume égal d'eau distillée et d'acétate d'éthyle ;
b) à laisser la phase d'acétate d'éthyle se séparer de la phase aqueuse ;
c) à séparer la phase d'acétate d'éthyle de la phase aqueuse ;
d) à ajouter le volume d'acétate d'éthyle de l'étape a) et à mélanger avec la phase aqueuse de l'étape c) ;
e) à laisser la phase d'acétate d'éthyle se séparer de la phase aqueuse ;
f) à séparer la phase d'acétate d'éthyle de la phase aqueuse ;
g) à répéter les étapes d) à f) ;
h) à éliminer l'eau des phases d'acétate d'éthyle ;
i) à ajouter de l'acétone à la phase d'acétate d'éthyle de l'étape h) pour précipiter l'immunoglobuline de requin ; et
j) à séparer l'immunoglobuline de requin purifiée de la phase d'acétate d'éthyle.

**9.** Composition pouvant être obtenue par le procédé suivant l'une quelconque des revendications précédentes, destinée à inhiber la prolifération du virus de la leucémie murine de Rauscher ou la prolifération de cellules tumorales chez un mammifère.

**10.** Composition suivant la revendication 9, qui est destinée à être administrée par voie intraveineuse ou intramusculaire.

**11.** Composition suivant la revendication 9 ou 10, dans laquelle la tumeur est un sarcome ou une leucémie.

**12.** Composition suivant l'une quelconque des revendications 9 à 11, comprenant en outre un support ou stabilisant pharmaceutiquement acceptable.

**13.** Composition suivant la revendication 12, dans laquelle le stabilisant pharmaceutiquement acceptable est le maltose.